# EUROPEAN PATENT APPLICATION

(11) **EP 1 978 100 A1**
(43) Date of publication of application: **08.10.2008**
(21) Application number: 07105535.4
(22) Date of filing: 03.04.2007
(51) Int. Cl.: C12P 3/00

(54) **Microbiologically induced carbonate precipitation**

(71) Applicant: Stichting GeoDelft, 2628 CK Delft (NL)
(72) Inventor: van Paassen, Leon Andreas, 2613 WD Delft (NL); Daza Montano, Claudia Mercedes, 1816 EH Alkmaar (NL); Kleerebezem, Robbert, 2611 NE Delft (NL); van Loosdrecht, Mark Cornelis Maria, 2548 AJ Den Haag (NL); van der Star, Wouter Roelof Lambertus, 2523 JE Den Haag (NL); van der Zon, Wilhelmus Hendrikus, 2521 ZL Den Haag (NL)
(74) Representative: van Loon, C.J.J.

(57) **Abstract**

The present invention relates to a method of forming a carbonate precipitate in a porous material, by a microbiologically induced reaction, comprising providing the material with a liquid substrate comprising an microbiologically oxidisable organic carbon source, an electron acceptor and a counter ion for carbonate, microbiologically oxidising the organic carbon source in an aqueous environment thereby forming carbonate, and precipitating the carbonate.

## Description

The invention relates to a method for forming a carbonate precipitate in a porous material, such as a soil formation or other geological formation. The invention further relates to a material obtainable by such method. The invention further relates to the use of a denitrifying micro-organism and/or a sulphate reducing micro-organism.

It is known that specific naturally present bacteria, namely urease containing bacteria, can cause biomineralisation in natural settings. Based upon this knowledge, the use of bacterial urease to modify a porous medium has been described by Nemati and Voordouw in Enzyme and Microbial Technology 33 (2003), page 635-642.

It has been contemplated that bacteria may be used without having to isolate the urease from the bacteria.

In "Ca-carbonates precipitation and limestone genesis -- the microbiogeologist point of view." Sedimentary Geology 126(1-4), Castanier, et al. (1999) try to explain observed natural occurrences of limestone. They propose several ways in which carbonate precipitation may theoretically occur including (i) abiotic chemical precipitation from saturated solutions; (ii) skeleton production by eukariotes; (iii) lowering of CO₂ pressure under effect of autotrophic processes; (iv) fungal mediation; and (v) heterotrophic bacterial mediation. Various possible metabolic pathways are taken into consideration, such as non-methylotrophic methanogenesis, anoxygenic photosynthesis, oxygenic photosynthesis, the nitrogen cycle and the sulphur cycle. For the nitrogen cycle several pathways are given, namely the ammonifaction of amino-acids, the dissimulatory reduction of nitrate, and the degradation of urea or uric acid.

However it is not described by Castanier, et al. how to apply, stimulate and/or control this metabolic activity in a porous material in order to change a property of a material by using a substrate comprising specific reactive components. In particular, it is not taught to introduce an oxidisable organic carbon source, an electron acceptor and a suitable counter ion for carbonate precipitation into a porous material to form a carbonate salt in the material, in order to change a property such as strength of the material or permeability of the material.

US 5,143,155 relates to a process to reduce the permeability of a subsurface geological formation by mineral precipitation, consisting of providing to the formation a micro-organism culture having the capability to sustain metabolic activity within the environment of the formation, injecting an aqueous mineralisation medium into the formation, and precipitating mineral matter from the mineralisation medium. The micro-organisms disclosed in this publication are urease producing bacteria or spores thereof.

WO2006/066326 relates to a method of forming a high strength cement in a permeable starting material, the method comprising the step of combining the starting material with effective amounts of (i) a urease producing micro-organism; (ii) urea; and (iii) calcium ions and wherein the effective amount of the urease producing organism provides a urea hydrolysis rate, under standard conditions, of 0.5-50 mM urea hydrolysed/min.

A drawback of using urease to form a carbonate mineral is the formation of high amounts of ammonia (typically 2 mole of ammonia is formed for each mole of carbonate that is formed). Ammonia is undesirable from an environmental point of view. Removal of the ammonia from the material or an effluent thereof (in case the material is flown through with a substrate for the bacteria) may be required or at least desired. Such removal may be difficult, in particular if the treated material is a geological formation.

It is an object of the present invention to provide a novel method for forming a carbonate precipitate in a porous material, in particular in a geological material, such as sand, clay, silt, loess *etc.,* or a composition comprising such material.

It is in particular an object of the invention to provide a method for forming a carbonate precipitate in a material, wherein the product of an undesired by-product such as ammonia is avoided or at least reduced.

One or more other objects which may be realised in accordance with the invention are apparent from the remainder of the description and/or claims.

It has now been found possible to realise one or more of such objects by forming a carbonate precipitate in a porous material, using a specific micro-organism and a specific substrate.

Accordingly, the present invention relates to a method of forming a carbonate precipitate in a porous material, by a microbiologically induced reaction, comprising providing the material with a liquid substrate comprising a microbiologically oxidisable organic carbon source, an electron acceptor and a counter ion for carbonate, microbiologically oxidising the organic carbon source in an aqueous environment thereby forming carbonate, and precipitating the carbonate.

The oxidisable organic carbon source, electron acceptor and counter ion may be applied to the material in a single liquid substrate. It is also possible to apply one or more components separately, such that oxidisable organic carbon source, electron acceptor and counter ion are combined in the material to form the liquid substrate comprising each of the components.

The term "or" as used herein means "and/or" unless specified other wise.

The term "a" or "an" as used herein means "at least one" unless specified other wise.

When referring to a moiety (*e.g.* a compound, an ion, an additive etc.) in singular, the plural is meant to be included. Thus, when referring to a specific moiety, *e.g*. "ion", this means "at least one" of that moiety, *e.g*. "at least one ion", unless specified otherwise.

In accordance with the invention one or more micro-organisms are used that oxidise one or more organic carbon sources, in an aqueous environment. As an oxidation product, carbon dioxide is formed. At least part of the carbon dioxide reacts with water to form carbonate. At least part of the carbonate is usually precipitated with a suitable counter ion, to form a solid mineral.

In accordance with the invention one or more redox reactions are induced in an aerobic, an anoxic or an anaerobic environment.

In an aerobic environment oxygen can be used as the (main) electron acceptor. An aerobic environment may in particular exist in a dry porous material, or in a wet porous material that is only partially saturated with water, wherein the degree of saturation is so low that that sufficient oxygen is present in the pores (or at least the permeability to oxygen is sufficiently high, to allow the oxygen to be added from outside the material), such that the microbiological oxidation can be fully or at least for a considerable part be effected by reduction of oxygen.

In particular in an anoxic or anaerobic environment, such as in a porous material essentially fully saturated with water or partially saturated with water, wherein the degree of saturation is so high that insufficient oxygen is present in the pores and the permeability to oxygen is insufficient to allow sufficient oxygen to be added from outside the material, one or more other inorganic or organic electron acceptors may still be used, such as one or more electron acceptors selected from the group of nitrate, nitrite, sulphate, sulphite, sulphur, H₂S, Fe³⁺ and Mn4+.

In some anaerobic environments, an electron acceptor which can be used is already present in the porous material, *e.g.* Fe³⁺ or Mn⁴⁺ or the oxidized forms of other metals or other solid or liquid materials may be naturally present. Of course, these may be added to the material, if desired.

In accordance with the invention it is possible to provide a porous material, in particular a material from geological origin or a geological information, with a carbonate precipitate, without forming an unacceptable amount of an undesired by-product, which may be undesired from an environmental point of view or which may cause a detrimental effect, such as inactivation or even killing of a micro-organism, due to toxicity of the by-product or an excessive increase in pH. In particular the invention may be carried out without forming a substantial amount of ammonia.

In particular, the present invention is advantageous in that the method is suitable to provide a clean porous material with a carbonate precipitate, without having to remove a waste-product, such as ammonia, from the material. The term "clean" as used herein in particular means "free of an unacceptable level of undesired by-products of the process", more in particular "essentially free of such by-products". Especially, a method of the inventions is clean in that it allows preciptation of carbonate without forming a substantial amount of ammonia. Thus, the invention also provides a simplified process compared to the use of certain micro-organisms, in particular urease forming micro-organisms.

A method of the invention may in particular be used as a biomineralisation method, in particular for treating a soil formation or another geological formation. More in particular, the method may be used to increase the mechanical strength of a porous material, to stabilise a (particulate) porous material and/or to reduce the permeability of a porous material to a liquid, such as water or oil, and/or a gas, such as air, oxygen or a hydrocarbon gas (such as methane).

A method of the invention may in particular be used to decrease chemical and/or mechanical erosion potential of a porous material, i.e. the amount of material which is removed in a specific period of time.

The present invention may be applied to produce cement for use in civil engineering, mining, erosion control, an environmental application or the manufacture of a special material. Ground improvement applications are particularly suited for the present invention. Civil engineering applications include application of the method to retaining walls, embankments (e.g. railway embankments, dams) and ground for tunnelling to reinforce and stabilise soil; foundations to improve skin friction of piles (bonding them to the "far field"); increasing the end-bearing capacity of piles; stiffening in situ formation to reduce pile design lengths; improving the bearing capacity of soils for non-piled foundations; the stabilisation of sands in earthquake zones at risk of liquefaction; preventing erosion by external environmental forces, such as wind and water or other dynamic impacts on the material; preventing erosion by internal forces, such as liquid or gas flow through the porous material.

A method of the invention may also be used in pavements to create "instant" pavements by surface treatment of natural or prepared sand surfaces for roads, runways etc and to rapidly repair degraded pavement sub base.

A method of the invention may also be used to preserve, restore, strengthen and protect weathered mortar and masonry in buildings such as heritage structures; consolidate and conserve decaying lime plasters in mural paintings; create architectural features in gardens and replicate ornaments in synthetic sandstone/limestone.

Uses in the mining industry include the use of the method to provide support to broken ground during tunnelling and mining; strengthen tailings dams to prevent erosion and slope failure; provide a permeable reactive barrier to allow drainage and remove acidic and heavy metal discharges from mines; bind dust particles on exposed surfaces to reduce dust levels; increase resistance to petroleum borehole degradation during drilling and extraction; increase the resistance of offshore structures to erosion of sediment within or beneath gravity foundations and pipelines.

It is further possible to use a method for manufacturing a construction material, such as stones, bricks, tiles *etc..*

The invention may also be employed in an environmental application such as the stabilisation and/or removal of pollutants (*e.g*. heavy metals, fibres, radioactive elements) from the environment by binding them into a carbonate (*e.g.* calcite) crystal structure or the control of erosion in a coastal area, a river bank, a lake side or the like by strengthening exposed surfaces and thus protecting areas subject to erosion.

Other uses include the creation of filters such as water filters and bore hole filters, and the immobilisation of bacterial cells and their enzymes into a cemented active biofilter.

The present invention offers versatile methodology. By choosing particular conditions, such as the choice of one or more micro-organisms to be used for carbonate formation, one or more electron acceptors and one or more carbon sources (which act in this case also as electron donor), one or more counter ions for precipitation of carbonate anions and/or one or more other factors, as described below, a property of the precipitated carbonate (mineral) can be influenced. By changing one or more of such conditions, it is for instance possible to alter the carbonate formation and/or precipitation rate. A change in such rate may not only be used to affect the total duration of the method, but also to influence, for instance, a crystal property of the precipitated crystal. A high carbonate formation and/or precipitation rate may thus be desired for obtaining a particular amount of calcium precipitate (crystals) within a short time-span. It is contemplated that a slow carbonate formation and/or precipitation rate may be advantageous to obtain a carbonate precipitate (crystal) with a different resulting material property, for instance enhanced density (reduced permeability/porosity) and/or enhanced strength.

The material usually is a material, that contains a fraction that is solid under ambient conditions (the conditions under which the method is carried out) and a fraction of pores, which may contain liquid and/or gas. A material is considered porous if it can be provided with, the carbon source, the electron acceptor and the counter ion. Thus, a low porosity (Vpores/Vtotal), such as a porosity of about 0.1 % or more is generally sufficient. In practice, in particular in case the material is a a geological formation or directly obtained there from, the porosity is usually higher, such as at least 5 %, at least 10 %, at least 20 % or at least 30 %. The porosity may be relatively high, *e.g*. 90 % or more. In practice, in particular in case the material is a geological formation or directly obtained there from, the porosity is usually lower, such as 50 % or les or 40 % or less.

A material, in particular a geological formation, preferably has a minimum permeability to water of 10⁻¹⁵ m/s. Such permeability is, *e.g.*, often found for foil constructions around soil bodies. In a particularly preferred embodiment, the permeability is at least 10⁻¹³ m/s. Such permeability is, *e.g*., often found for concrete. More in particular the permeability may be at least 10⁻⁹ m/s. Such permeability is, *e.g*., often found for clay layers.

The permeability may be 10⁻⁶ m/s or higher, e.g. as found in a sand layer, or 10⁻² m/s or higher, *e.g*. as found in a gravel layer.

In particular any material that is permeable to the carbon source, the electron acceptor and the counter ion (*i.e*. any material that has voids that are sufficiently large to let liquid comprising these components, which are usually dissolved in the liquid pass through it, at least under pressure) may be used. In case it is desired to use a micro-organism to the material that is not naturally present, for an *in situ* activity such as biomineralisation, the material should usually also be permeable to the micro-organism to allow introduction of the micro-organism.

In particular, a material to be used in a method of the invention may be a soil formation or another geological formation. The method is also suitable to treat another mineral material, such as a mineral material at least partially originating form a geological formation. The geological formation may in particular be a body, comprising sediment (clay, silt, loess,sand and/or gravel), sedimentary rock, soil material and/or rock material with permeability e.g. fractured rocks. Preferably, the solid material is a soil formation, another geological formation or a material at least partially originating from soil or another geological formation selected from the group of formations comprising sand, in particular sand dunes, formations comprising clay, formations comprising silt, formations comprising loess, formations comprising limestone, formations comprising sandstone, formations comprising gravel, formations comprising rock material permeable to bacteria (such as fractured rocks) and material at least partially originating from any of these formations.

Particular suitable rock materials include sedimentary rock such as a terrigenous, chemical/biochemical or organic sedimentary rock, such as selected from the group of sedimentary rocks comprising: clastic sedimentary rock, such as conglomerate, breccia, sandstone, siltstone and shale or non-clastic sediments such as limestone, gypsum, dolostone, peat and lignite.

Particular suitable materials at least partially originating from a soil formation or other geological formation include tailings, such as mine tailings, and sludges.

The material may also be a geological formation or a material originating there from, comprising a substantial amount of organic matter, such as peat. The peat may be a peat stratum, peat bog or peat moor.

Further, a method of the invention may be used to form a carbonate precipitate in a material such as plaster, sediments sawdust, cardboard, particle board, mortar or soft wood.

Preferably, the material to be treated is a particulate material, which may be unconsolidated or at least partially consolidated. Especially, in such particulate material at least 50 vol % of the material, at least 90 vol % of the material or at least 95 vol % of the material may have a particle size of 0.001-64 mm, i.e. wherein at least 50 vol %, at least 90 vol % or at least 95 vol % of the material passes through a sieve with a mesh size in the range of 0.001-64 mm. In particular, least 50 vol %, at least 90 vol % or at least 95 vol % of the material may have a size of at least 0.0625 mm or at least 2 mm. In particular, least 50 vol %, at least 90 vol % or at least 95 vol % of the material may have a size of up to 2 mm or up to 0.0625 mm.

For instance, a material other than a geological formation (as such), in particular such material in a particulate form, may be used to make a composite of the material embedded in the carbonate precipitate. Such composites can find use as a construction material. It can for instance be a stone, plate, composite bar, tile, *etc.*

In principle, in accordance with the invention any micro-oganism capable of oxidising the carbon source in the presence of the electron acceptor may be used. Such an organism may be found in a geological formation or material at least partially originating there from. The micro-organism may be selected from a strain library. The micro-organism may be isolated or enriched outside the material or *in situ* using a selective cultivation procedure.

In a preferred method of the invention at least one denitrifying micro-organism and/or at least one sulphate reducing micro-organism is used. Such an organism may be found in a geological formation or material at least partially originating there from. In particular, it has been found that a suitable micro-organism may be found in a natural soil, like beach sand - such as sand found at a North Sea coast - or in garden soil. Further a suitable micro-organism may be found in an artificial environment, like in sludge from a wastewater treatment plant.

In an embodiment, one or more denitrifying micro-organisms are selected from the group of members capable of denitrification, such as denytrifying species from the bacterial genera : *Bacillus, Jonesia, Rhodobacter, Rhodopseudomonas, Rhodoplanes, Beggiatoa, Thiobacillus, Thioploca, Ralstonia, Paracoccus, Pseudomonas, Nitrobacter, Nitrosomonas, Zavarzinia, Aquaspirillum, Hyphomicrobium, Empedobacter, Azospirillum, Alteromonas, Alcaligenes, Azoarcus, Bradyrhizobium, Sinorhizobium, Aquifex, Thermothrix, Halomonas. Chromobacterium, FlavobacteriumBlastobacter, Hyphomicrobium, Cytophaga, FLexibacter* and/or *Magnetospirillum*

In an embodiment, a denitrifying *Archaea and*/*or Eukarya* is used.

In an embodiment, a suitable sulphate and/or sulphur reducing micro-organism is selected from the group of members capable of sulphate and/or sulphur reduction from any of the genera: *Desulfovibrio, Desulfomicrobium, Desulfobotulus, Desulfofustis, Desulfotomaculum, Desulfumonile, Desulfobacula, Archeoglobus, Desulfobulbus, Desulforhopalus Thermodesulfobacterium, Desulfobacter, Desulfobacterium, Desulfococcus, Desulfonema, Desulfosarcina, Desulfoarculus, Desulfacinum, Desulforhabdus, Thermodesulforhabdus, Desulforomonas, Desulfurella, Sulfurospirillum andlor Campylobacter.*

In an embodiment, a sulphate and/or sulphur reducing *Archea and*/*or Eukaria* is used.

The micro-organism preferably is vegetative, because such organism is directly active, although in principle spores can be used, which first have to be activated into a vegetative micro-organism.

In particular in case the material is a soil formation, another geological formation or a material at least partially originating from such material, a micro-organism may be used which is indigenous to the material. Thus, the invention makes it possible to microbiologically form a carbonate precipitate without needing to introduce the micro-organism into the material. This further simplifies the method.

The micro-organism concentration is not particularly critical, as in accordance with the present invention it is possible to allow micro-organisms to grow *in situ* in the material, while performing the red ox conversion and the precipitation reaction. For a desired amount of carbonate to be formed within a reasonably short time-span, an active (initial) biomass concentration of 10 mg/L (0.001 C-mol/L) or more is preferred (based on grams of volatile suspended solids (VSS) per litre of the total volume of the material). An (initial) biomass concentration of 500 mg/L up to 5000 mg/L (0.05 - 0.5 C-mol/L) is particularly preferred for a high rate of carbonate formation. *E.g.,* in an embodiment, at an initial biomass concentration of 500 mg/L a typical production rate of at least about 25mM/day of carbonate precipitate has been found, using one or more calcium salts of one or more deprotonised organic acids as source containing the carbon source and with calcium nitrate as source containing the electron acceptor.

In a method wherein a micro-organism is to be introduced into the material to be treated, use may be made of a micro-organism culture obtained by growing micro-organisms from a suitable source such as a geological formation comprising suitable micro-organisms, such as (beach) sand.

The total concentration of one or more electron acceptors may be chosen within a wide range. Usually, the concentration is chosen high enough to exceed the solubility of the product of the envisaged precipitation of produced carbonate and avoid limitation for the oxidation reaction, but low enough to prevent precipitation of the reagents and nutrients and toxicity or inhibition of the metabolic reactions of the micro-organisms.

In a preferred method, in particular in a method using denitrification, the total concentration of one or more electron acceptors usually is at least 0.001 M, preferably at least 0.01 M and in particular at least 0.1 M. The total concentration of one or more electron acceptors usually is up to saturated conditions, preferably up to 1 M and in particular up to 0.5 M or 0.25 M. A relatively high concentration is preferred for a high reaction rate, the possibility to treat material over a larger distance, and thus for a reduced treatment time to achieve a specific level of carbonate precipitate formation in a specific volume of material. In practice, the upper limit is generally defined by the solubility of the least soluble added component in the liquid substrate (carbon source, electron acceptor and counter ion). The lower limit is generally defined by the solubility product of the produced carbonate with the precipitating counterion, i.e. if product concentration is too low no precipitate will be formed.

Further, a relatively low concentration may be desired in view of the viability of the micro-organism. In particular, a too high salt concentration can be toxic or inhibiting for the micro-organism or can cause the need for a more salt tolerant (halophilic) micro-organism, more tolerant to a high salinities of a high electron acceptor concentration, making the method less simple.

One or more electron acceptors in the oxidation reaction with the carbon source may be selected depending upon the micro-organism used to oxidise the carbon source. Particularly suitable is an electron accepting anion. Preferably such anion is a compound comprising one or more oxygen atoms. Such anion may also serve as an oxygen source for the carbonate.

In particular in case a denitrifying micro-organism is used, nitrate and/or nitrite may be used as an electron acceptor.

The use of nitrate and/or nitrite (in combination with a denitrifying micro-organism) is in particular advantageous with respect to the fact that in general a harmless by-product is formed (nitrogen gas). Thus, such method in particular qualifies as a clean and/or environment-friendly method.

Further, it is apparent that in a method of the invention denitrifying bacteria can grow well, which contributes to an advantageous process time to achieve a specific degree of carbonate production.

Further, it is apparent that the oxidation of the carbon source may take place relatively fast compared to a method wherein another micro-organism is used. Thus, an intended effect, such as desired degree of biomineralisation, may be accomplished relatively fast, for instance within 100 days, or within 50 days.

On the other hand the oxidation rate is low enough to avoid formation of gas (in particular N₂ and/or some gaseous CO₂, as opposed to dissolved CO₂ at an excessive rate. It is contemplated that a very high oxidation rate, as may occur in at least some prior art processes, may result in an excessive gas production rate, which can counteract the intended effect of soil improvement as the gas may build up pressure or try to escape to the surface by forming cracks or loosen the material, especially in a shallow treatment close to the surface of the material Accordingly, a very high oxidation rate, as in at least some prior art processes, may result in a reduced efficiency in terms of improvement, in particular in case the material is a soil.

In particular in case a sulphate reducing micro-organism is used, sulphate and/or or sulphite may be used as an electron acceptor. It is contemplated that the oxidation of the carbon source in a method wherein sulphate, sulphur and/or sulphite is used (in combination with a sulphate reducing micro-organism) is usually relatively slow. Further, complete reduction of sulphate and/or sulphite also causes the production of sulphide or hydrogensulphide. The sulphide and/or hydrogensulphide can optionally precipitate with a suitable counter ions.

In accordance with the invention, carbonate is usually precipitated with a suitable counter ion (a cation). In particular suitable is a cation having a sufficient solubility in the liquid substrate, to avoid precipitation with one or more other components in the liquid substrate, before formation of carbonate and a relatively poor solubility with carbonate. The skilled person will be able to determine suitable solubilities based on the information described herein, optionally in combination with common general knowledge and/or some routine testing.

Preferred examples of counter ions are alkaline earth metal ions, iron ions, aluminium ions, manganese ions and other cations having a similar or higher solubility in the substrate in the absence of carbonate and a similar or lower solubility with carbonate. The counter ion may in particular be added as a salt (solution) of an electron accepting anion, such as a nitrate salt or a sulphate salt. In addition or alternatively, the counter ion may be added as a salt of the carbon source, such as a salt of an organic acid, such as an acetate salt or a lactate salt.

It is also possible to add at least part of the counter ion(s) in the form of another salt, in particular as a chloride salt, as such salts in general have a good solubility. Preferably, if such salt is used, it forms a minor part of the source for the counter ion(s), in order to keep the formation of one or more undesired by-products low or to avoid.

An alkaline earth metal ion, in particular calcium and/or magnesium ions, is particularly preferred because of its low solubility with carbonate

Counter ions which can occur in more than one valence state, such as iron ions or manganese ions, may be used in any ionised state.

One or more ions such as iron ions, manganese ions, may be used in one ore more ways: these may be used in their fully oxidized state, *e.g.* Fe³⁺, Mn⁴⁺ as electron-acceptor and/or in their partially reduced state, *e.g*. Fe2⁺, Mn²⁺, as counter ion in the precipitation of carbonate.

For instance, FeCO₃ has a low solubility constant, which is advantageous in that it readily precipitates, also at a relatively low concentration and/or relatively low pH.

Further, the use of iron may be in particular advantageous in case the method of the invention encompasses the use of sulphate in combination with a sulphate reducing micro-organism, as it may be used to precipitate iron sulphide, formed by the micro-organism. Thus, the release of H₂S in the environment may be reduced or essentially avoided.

The counter ion concentration is chosen such that when sufficient carbonate has formed, the counter ion precipitates with the carbonate ion. The skilled person will be able to determine suitable concentrations based on the equilibrium constant for precipitation (Kₛₚ)under the existing conditions. As the skilled person knows the Kₛₚ is temperature dependent. As a guideline, the following values can be taken into consideration:

| Mineral | Kₛₚ (at 10°C) |
|---|---|
| MgCO₃ (magnesite) | 3.5 x 10⁻⁸ |
| CaCO₃ (calcite) | 3,4 x 10⁻⁹ |
| FeCO₃ (siderite) | 3,2 x 10⁻¹¹ |

Usually the total counter ion concentration is at least 0.001 M, in particular at least 0.01 M more in particular at least 0.1 M. Usually the total counter ion concentration is up to saturation, in particular up to 1 M, more in particular up to 0.5 M or 0.25 M. In principle, the higher the counter ion concentration, the more easy the precipitation of carbonate is induced and the more efficient the precipitation takes place (higher rate, lower pH, lower temperature, less loss of CO₂). If the counter ion concentration is higher than or equal to the amount of CO₂ produced, a particularly highest efficiency is achieved. On the other hand, if concentration is excessively high, the counter ion might become toxic to the micro-organism, or it can inhibit its activity.

The organic carbon source may in principle be any oxidisable carbon source which can be oxidised by the micro-organism. For instance, the carbon source may be a (mixture of) organic compound(s) in or from a waste stream, which are not yet (fully) oxidized, such as in or from a waste stream from a digester.

Usually, the organic carbon source is selected from organic carbon containing energy sources for a micro-organism that is used. For instance, the source may be selected from organic acids, alcohols, carbohydrates, amino acids and peptides (including polypeptides and proteins). In a preferred method at least one organic carbon source is present selected from the group of organic acids, alcohols, monosaccharides and disaccharides. Such compounds are readily available. Further, these generally consist of C, H and O, and as a result, the metabolic products are usually non-detrimental to the environment and/or the micro-organism. In particular an organic acid or an alcohol may be preferred for a high rate of forming carbonate. Good results have in particular be achieved with an organic acid. As used herein the term organic acid includes the free acid, partially deprotonated acid and fully deprotonated acid. Analogously, when referred to a specific deprotonated anion of an acid (*e.g*. acetate, formate, *etc*.) this term is intended to include the free acid as well (*e.g.* acetic acid, formic acid. *etc*.). Thus, the organic acid may be administered as a salt, for instance an alkali metal salt and/or as a salt of one or more counter ions for the carbonate.

In a particular preferred method at least one carbon source is present selected from the group of acetate, butyrate, citrate, glyoxalate, formate, lactate, malate, malonate, oxalate, propionate, succinate, tartrate, ethanol, glycerol, methanol, n-propanol, iso-propanol, glucose and fructose.

Different carbon sources may offer different advantages.

For instance, a C1-C4 alcohol, monosaccharides and some organic acids, notably formate, citrate, lactate, acetate are highly soluble in water, which is advantageous because it can be made available at a high concentration to a micro-organism in the material.

Malonate, glyoxilate, acetate, tartrate, malate and in particular oxalate and formate have shown a high carbonate production rate (at a specific micro-organism mass) compared to some other carbon sources, notably a monosaccharide such as glucose.

Acetate, propionate, malonate, glycerol, glyoxilate, acetate, tartrate, malate, butyrate, citrate and in particular lactate, succinate and glucose have shown to be particularly beneficial with respect to contributing to the growth of micro-organisms, which may contribute to increased carbonate production at a later stage of the method of the invention.

Formate, and in particular acetate and malate are advantageous in that these have a high solubility with one or more counter ions, in particular with calcium.

For a fast process, in particular good results have been achieved with at least one carbon source selected from acetate, lactate and formate.

In addition to the above mentioned components one or more other components may be administered. In particular one or more (further) nutrient components for the micro-organism, one or more pH adjusting agents (acids/bases) to adjust the pH to a desired levels and/or one or more buffer components to maintain the pH within a desirable range, may be added.

Preferably at least a sulphur, phosphorus and/or iron source are present. The skilled person will know to select suitable nutrients and concentrations based on the specific conditions in the material (such as a desire for one or more added nutrient components for specific micro-organisms found in or added to the material, natural presence of specific nutrients in the material, e.g. iron (salt/oxide), phosphate)

The method may be carried out at acidic, neutral or alkaline pH.

In particular, the pH usually is up to 4 units below or above neutral pH, preferably up to 2 units below or above neutral pH, in particular up to 1.0 unit below or above neutral pH, more in particular in particular up to 1.0 unit below or above neutral pH. Further, the nature of the micro-organisms may determine a desirable pH. On the other hand, a specific pH may be choosen to create an environment wherein one or more specific micro-organisms of which a high activity is desired are thriving and/or wherein bacteria of which a high activity is undesired, show a low activity, or become inactivated.

In general, a micro-organism used in accordance with the invention thrives well at a pH within the above indicated range. In particular in case an alkaliphile is used, a relatively strongly alkaline pH may be particularly suitable.

In view of the above, the pH of an aqueous liquid comprising one or more components, such as an electron accepting anion, a carbon source and/or a counter ion for the carbonate (measured at 20 °C) is usually at least 3, preferably at least 5, in particular at least 6, more in particular at least 6.5, even more in particular at least 7.0.

In view of the above, the pH of an aqueous liquid comprising one or more components, such as an electron accepting anion, a carbon source and/or a counter ion for the carbonate (measured at 20 °C) is usually up to 11, preferably up to 9, more preferably up to 8, in particular up to 7.5.

A method of the invention may be carried out within a wide temperature range. In view of administering the needed components to the material, which is usually accomplished by using an aqueous liquid comprising one or more of the components, the temperature preferably is at least 0 °C. Depending upon the micro-organism the optimum temperature may be different. Further, a high temperature may be advantageous for realising a high solubility of one or more components used in the method and/or a high reaction rate. In particular the temperature may be at least 10 °C, at least 20 °C, at least 30 °C or at least 40 °C. For micro-organisms that are hyperthermophiles a higher temperature may be preferred, such as a temperature of at least 60°C, at least 80 °C or at least 90 °C. The upper limit is determined by what is suitable for a micro-organism to be used. Further, a relatively low temperature may be beneficial in that the solubility of a carbonate salt usually is lower at a low temperature and thus a larger fraction of the formed carbonate is precipitated. Thus a preferred temperature may be up to 120 °C, up to 90 °C, up to 60 °C, up to 40 °C or up to 30 °C.

Regarding the dosage of different components used in accordance with the invention the following is observed.

As indicated above, a method of the invention may be carried out without adding an micro-organism to the material, in particular in case of a geological formation or material there from, in case such material already indigenously comprises a suitable micro-organism. It is also possible to administer a suitable micro-organism (culture) to the material. If this is done, it is usually sufficient to administer the micro-organism, in an initial phase of the method, until a desired initial biomass concentration (or activity) is obtained, although in principle it is also possible to continuously or intermittently administer a micro-organism.

Suitable methods are known in the art, for instance mixing (in case the material is of a particulate nature and not part of a geological formation) or injection, *e.g.* as described in US 5,143,155. WO 2006/066326 also describes suitable methods.

In particular, in case the micro-organism is introduced into the material, it may be advantageous to immobilise the micro-organism. Immobilisation may be accomplished by
forming a first zone comprising the micro-organism in the material and allowing the first zone to move through at least part of the material; forming a second zone comprising an effective amount of a flocculating agent to the material and allowing the second zone to move through at least part of the material,
wherein the zones are allowed to move such that the zones become at least partially overlapping and at least part of the micro-organism flocculate, thereby becoming immobilised.

Suitable and preferred conditions and agents for such immobilisation are described in the non-prepublished PCT/NL2006/000598, of which the contents are enclosed herein by reference, and in particular the parts with respect to the conditions and agents mentioned in page 13, line 1 to page 14, line 3, and/or page 14, line 8 to line 20. A suitable way to move the zones in the material may also be based on known methodology for introducing inorganic solutions into and moving them through the material. See *e.g.* WO 00/31209.

With respect to applying the components other than the micro-organism (in particular electron accepting anion, organic carbon source, counter ion for carbonate), these may be administered together or separately. The administration regime is preferably such that the concentration is substantially maintained within a desired concentration range. One or more of these components may be administered continuously or intermittently.

In as far as the components need to be applied to the material, this may be accomplished in a variety of ways. One or more of the components may be forced into the material under pressure such as by flushing or injection; one or more of the components may be sprayed, dripped or trickled onto or into the material. Depending on the size and form of the starting material, the material may be soaked with or immersed in the liquid substrate.

The absolute pressure may be chosen within wide ranges, depending upon the nature of the material and/or -in case of a geological formation or a subterranean artificial structure - the depth at which the material to be treated is situated. If pressure is used, the absolute pressure may in particular be between 0.1 and 100 bar. Suction may be used (absolute pressure of less than 1 bar, for instance at least 0.1 bar). Alternatively the pressure may at least 1 bar or at least 2 bar. The pressure may be up to 3 bar or up to 10 bar, which is in particular sufficient for relatively shallow subsurface applications or for applications that are not sub-surface. In particular for a relatively deep subsurface application a pressure of up to 50 bar or up to 100 bar may be used.

A method of the invention may also be repeated at least once in order to achieve a particular intended result, such as a particular strength or impermeability. It will be appreciated that when the method is repeated to gain incremental increases in strength, not all of the reagents need to be added each time.

The invention will now be illustrated by the following examples.

### Example 1

Experiments were performed to show that micro-organisms can oxidise organic compounds by denitrification and simultaneously grow and induce precipitation of calcium carbonate. The following aqueous solutions, containing the carbon source/electron donor, the electron acceptor (NO3-) and counterion (Ca²⁺), were prepared:
1. Calcium acetate 11 mM, calcium nitrate 9 mM
2. Potassium acetate 50 mM, calcium nitrate 20 mM
3. Potassium citrate 23 mM, calcium nitrate 20 mM

To all solutions additional nutrients were added to avoid nutrient limited growth of the micro-organisms, comprising 0.003 mM (NH₄)₂SO₄, 0.0024 mM MgSO4.7H2O, 0.006 mM KH₂PO₄, 0.014 mM K_{2H}PO₄ and 1ml/L trace element solution. All solutions were inoculated with a few grams of garden soil.

All three solutions showed precipitation, gas formation and microbial growth, within several days (1 day on citrate and 7 to 10 days on acetate), with the pH remaining within 1 unit from neutral

### Example 2

Experiments of Example 1 were repeated to show that microbial activity and growth and carbonate precipitation are possible using different inocula. Aqueous solutions were prepared according to Example 1. The solutions were inoculated with solution 3 from Example 1, beach sand and activated sludge from a municipal wastewater treatment plant with denitrifying activity. All inocula showed precipitation, gas formation and microbial growth.

### Example 3

The experiments of Example 1 was repeated to show that microbial activity and growth and carbonate precipitation are also possible simultaneously at higher calcium concentrations. Aqueous solutions were prepared with same acetate/nitrate-ratio as solution 1, in Example 1, but now with total Ca²⁺ concentration 50 mM, 250 mM and 500 mM. Additional nutrients were added according to Example 1. The solutions were inoculated using solution 3 of Example 1. All solutions up to 250 mM showed precipitation, gas formation and microbial growth within 14 days.

### Example 4

Experiments were performed firstly to show that microbial activity and growth, and carbonate precipitation can be induced in porous media and secondly to get an indication of reaction rates under several conditions in order to estimate the required time to reach a target amount of precipitate.

Bulk solutions were made comprising calcium nitrate and/or calcium or sodium salts of various organic acids: acetate, formate, lactate, malate and succinate and calcium nitrate dissolved in water. Concentrations of carbon source and electron acceptor were calculated according to the metabolic stoichiometry and based on equal amount of COD:
1. Calcium formate 150 mM, calcium nitrate 32 mM,
2. Sodium succinate 48 mM, calcium nitrate 24 mM,
3. Sodium lactate 50 mM, calcium nitrate 25 mM
4. Sodium malate 50mM, calcium nitrate 26 mM
5. Calcium acetate 38 mM, calcium nitrate 30 mM

Calcium chloride was added to the media to have a total dissolved calcium concentration of 200mM. Additional nutrients were added according to Example 1.

PVC tubes (length 20 cm, diameter 4,5 cm) were filled with beach sand and flown from bottom to top.The bulk solutions were flown through the column at a flow rate of 100 ml (approximately 1 pore volume) per day. Chemical Oxygen Demand (COD), NO₃⁻, NO₂⁻ and Ca²⁺ concentrations were measured on both influent and effluent.

All five columns showed precipitation, gas formation and microbial growth, within several days. From the difference between influent and effluent concentrations a conversion rate was determined, from which the time needed to reach a target precipitation of 40 kg Ca²⁺/m³ was estimated, for each of the carbon sources. The results are shown in the following table.

| carbon source | time to target [days] |
|---|---|
| Acetate | 38 |
| Lactate | 53 |
| Formate | 166 |
| Succinate | 96 |
| malate | 113 |

In each of the experiments it was found that the pH remained about 7 throughout the process. This indicates that no precautions are needed to avoid a detrimental change in pH causing micro-organism inactivation or lack of precipitation.

## Claims

1. Method of forming a carbonate precipitate in a porous material by a microbiologically induced reaction, comprising providing the material with a liquid substrate, comprising a microbiologically oxidisable organic carbon source, an electron acceptor and a suitable counter ion for carbonate precipitation, microbiologically oxidising the organic carbon source in an aqueous environment thereby forming carbonate, and precipitating the carbonate with the counter ion.

2. Method according to claim 1, wherein one or more denitrifying micro-organisms are used to oxidise the organic carbon source.

3. Method according to claim 1 or 2, wherein the carbonate is formed in the presence of at least anion selected from the group of nitrate and nitrite, in a total nitrate+nitrite concentration of 0.001 to 1 M, preferably of 0.01 to 0.5 M, in particular of 0.01 to 0.25 M.

4. Method according to any of the preceding claims, wherein one or more sulphate and/or sulphur reducing micro-organism are used to oxidise the organic carbon source.

5. Method according to any of the preceding claims, wherein the carbonate is formed in the presence of sulphate and/or sulphite, in a total sulphite+sulphate concentration of 0.001 M to 1M, preferably of 0.01M. to 0.05M.

6. Method according to any of the preceding claims, wherein a micro-organism is used that is naturally present in the material to be treated.

7. Method according to any of the preceding claims, wherein the carbonate is formed in the presence of at least counter ion selected from the group of multivalent metal ions and semi-metal ions, in particular from calcium ions, magnesium ions, iron ions, and manganese ions.

8. Method according to any of the preceding claims, wherein the total concentration of the counter ions is in the range of 0.001 M to saturated concentration in the liquid substrate, before formation of carbonate, preferably of 0.01 to 1 M, more preferably of 0.1 to 0.25 M

9. Method according to any of the preceding claims, wherein at least one organic carbon source is present selected from the group of organic acids, alcohols and carbohydrates.

10. Method according to claim 9, wherein at least one carbon source is present selected from the group of acetate, butyrate, citrate, glyoxalate, formate, lactate, malate, malonate, oxalate, propionate, succinate, tartrate, ethanol, glycerol, methanol, n-propanol, iso-propanol, glucose and fructose.

11. Method according to any of the preceding claims, wherein the carbon source, electron-donor and/or electron-acceptor are added as one or more salts of one or more counter ions, soluble in the liquid substrate at the used concentration.

12. Method according to any of the preceding claims, wherein the organic carbon source concentration is in the range of 0.001 M to saturated concentration in the liquid substrate, before formation of carbonate, preferably of 0.01 to 1 M, more preferably of 0.1 to 0.5 M, in particular of 0.1 to 0.25 M.

13. Method according to any of the preceding claims wherein the porous material is a soil formation, another geological formation or a material at least partially originating from soil or another geological formation, preferably a formation selected from the group of formations comprising sand, in particular sand dunes, formations comprising clay, formations comprising silt, formations comprising loess, formations comprising limestone, formations comprising sandstone, formations comprising gravel, formations comprising rock material permeable to bacteria (such as fractured rocks).

14. Method according to any of the preceding claims, wherein the pH is within 4 pH units from neutral pH, in particular within 2 pH unit from neutral pH, more in particular within 1 pH-unit from neutral pH.

15. Method according to any of the preceding claims, wherein the pressure is between 0.1 and 100 bar, in particular between 1 and 3 bar.

16. Method according to any of the preceding claims, wherein the temperature is between 0 and 100 degrees Celsius, in particular between 5 and 50 degrees Celsius, more in particular between 10 and 25 degrees Celsius.

17. Method according to any of the preceding claims, wherein the overall salinity of the fluid already present in the pore space prior to providing the liquid substrate is between 0 and a saturation, in particular between the salinity of fresh water (≈0.1g total dissolved solids/L) and seawater (≈35 g/L, mainly NaCl).

18. Method according to any of the preceding claims, wherein oxidation reaction takes place under aerobic, anoxic, or anaerobic conditions

19. Material obtainable by a method according to any of the preceding claims.

20. Use of a denitrifying micro-organism and/or a sulphate reducing micro-organism to increase the mechanical strength of a porous material.

21. Use of a denitrifying micro-organism and/or a sulphate reducing micro-organism to decrease erosion potential of a porous material.

22. Use of a denitrifying micro-organism and/or a sulphate reducing micro-organism to decrease the air permeability of a porous material.

23. Use according to claim 20, 21 or 22, wherein the material is a material as defined in claim 13.
